# EUROPEAN PATENT SPECIFICATION

(11) **EP 3 217 912 B1**
(45) Date of publication and mention of the grant of the patent: **24.07.2024**
(21) Application number: 15858832.7
(22) Date of filing: 12.11.2015
(51) Int. Cl.: A61B 34/00, A61B 34/35, A61B 34/10, A61B 34/37, A61B 34/20, A61B 90/00

(54) **INTEGRATED USER ENVIRONMENTS**
INTEGRIERTE BENUTZERUMGEBUNGEN
ENVIRONNEMENTS UTILISATEURS INTÉGRÉS

(30) Priority: 13.11.2014 US 201462079392 P
(43) Date of publication of application: 20.09.2017
(73) Proprietor: Intuitive Surgical Operations, Inc., Sunnyvale, CA 94086 (US)
(72) Inventor: JARC, Anthony M., Sunnyvale, California 94087 (US); CHAU, Joey, Cupertino, California 95014 (US)
(74) Representative: MacDougall, Alan John Shaw
(86) International application number: PCT/US2015/060298
(87) International publication number: WO 2016/077531

(56) References cited:
- WO-A1-2011/060171
- US-A1- 2003 144 649
- US-A1- 2003 216 715
- US-A1- 2011 238 079
- US-A1- 2014 176 533
- US-B1- 7 075 556
- US-B2- 7 907 166

## Description

### RELATED APPLICATIONS

This patent application claims priority to and the benefit of the filing date of U.S. Provisional Patent Application 62/079,392, entitled "INTEGRATED USER ENVIRONMENTS," filed November 13, 2014.

### FIELD

Embodiments described herein generally relate to network communications and in particular, to systems and methods for integrated user environments.

### BACKGROUND

Minimally invasive medical techniques are intended to reduce the amount of tissue that is damaged during diagnostic or surgical procedures, thereby reducing patient recovery time, discomfort, and deleterious side effects. Teleoperated surgical systems that use robotic technology (so-called surgical robotic systems) can be used to overcome limitations of manual laparoscopic and open surgery. Advances in telepresence systems provide surgeons views inside a patient's body, an increased number of degrees of motion of surgical instruments, and the ability for surgical collaboration over long distances. In view of the complexity of working with teleoperated surgical systems, proper and effective training is important.

US2014/176533 discloses methods and systems for role designation with multiple sources. A method for role designation can comprise rendering a common field of interest that reflects a presence of a plurality of elements, wherein at least one of the elements is a remote element located remotely from another of the elements, receiving a role designation, and updating the common field of interest based upon the role designation. The methods and systems can also be used to superimpose imaging data of the operative anatomy onto the anatomy itself for guidance and orientation (augmented reality). This document does not disclose that the local and remote instruments operated by the users of the first and second user interfaces are virtual instruments.

WO2011/060171 describes a minimally invasive surgical system. A hand tracking system tracks a location of a sensor element mounted on part of a human hand. A system control parameter is generated based on the location of the part of the human hand. Operation of the minimally invasive surgical system is controlled using the system control parameter. Thus, the minimally invasive surgical system includes a hand tracking system. The hand tracking system tracks a location of part of a human hand. A controller coupled to the hand tracking system converts the location to a system control parameter, and injects into the minimally invasive surgical system a command based on the system control parameter.

The present invention provides a method and system for managing a user interface as set out in the appended independent claims. Optional features are defined in the appended dependent claims.

### BRIEF DESCRIPTION OF THE DRAWINGS

In the drawings, which are not necessarily drawn to scale, like numerals may describe similar components in different views. Like numerals having different letter suffixes may represent different instances of similar components. Some embodiments are illustrated by way of example, and not limitation, in the figures of the accompanying drawings in which:
FIG. 1 is a schematic drawing illustrating a teleoperated surgical system, according to an embodiment;
FIG. 2A is a drawing illustrating a master assembly, according to an embodiment;
FIG. 2B is a drawing illustrating a master controller of a master assembly, according to an embodiment;
FIG. 2C is a drawing illustrating an armrest of a master assembly, according to an embodiment;
FIG. 3 illustrates a virtual surgical site, according to an embodiment;
FIG. 4 illustrates a process to composite two virtual surgical sites, according to an embodiment;
FIG. 5 is a data flow diagram illustrating cooperative data sharing between a trainee system and a proctor system, according to an embodiment;
FIG. 6 is a block diagram illustrating a master assembly, according to an embodiment;
FIG. 7 is a flowchart illustrating a method of scoring a teleoperated surgical training session, according to an embodiment; and
FIG. 8 is a block diagram illustrating a machine in the example form of a computer system, within which a set or sequence of instructions for causing the machine to perform any one of the methodologies discussed herein may be executed, according to an example embodiment.

### DESCRIPTION OF EMBODIMENTS

The following description is presented to enable any person skilled in the art to create and use systems and methods of a medical device simulator. Various modifications to the embodiments will be readily apparent to those skilled in the art, and the generic principles defined herein can be applied to other embodiments and applications without departing from the scope of the inventive subject matter. Moreover, in the following description, numerous details are set forth for the purpose of explanation. However, one of ordinary skill in the art will realize that the inventive subject matter might be practiced without the use of these specific details. In other instances, well-known machine components, processes and data structures are shown in block diagram form in order not to obscure the disclosure with unnecessary detail. Flow diagrams in drawings referenced below are used to represent processes. A computer system can be configured to perform some of these processes. Modules or subsystems within flow diagrams representing computer implemented processes represent the configuration of a computer system according to computer program code to perform the acts described with reference to these modules. Thus, the inventive subject matter is not intended to be limited to the embodiments shown, but is to be accorded the widest scope consistent with the principles and features disclosed herein.

### Introduction

Surgical training can come in various forms, including observation, practice with cadavers or surgical training models, and simulation training. In the field of teleoperated surgery, all of these training techniques can be used. In order to provide a consistent and repeatable experience, simulation training provides distinct advantages.

When analyzing performance for a teleoperated simulator, instructional objectives can be viewed on a continuum with basic system skills on one end of the continuum and robotic surgical procedures on the other end. In the middle, robotic surgical skills and tasks are represented. Thus a user can begin learning with basic robotic system skills, such as dexterous tasks like needle targeting, moving objects, or navigating instruments in space. Eventually, the user can progress to the middle of the continuum and practice robotic surgical skills, such as suturing or knot tying. After gaining proficiency in skills, the user can progress to robotic surgical procedures and procedural tasks, such as a hysterectomy.

Simulation training can be provided to a user in various modes. The user can participate in individual training modules attempting a training task with or without guidance. Such guidance can be provided by the training module, for example, with audio prompts, textual overlays, or the like. Alternatively, the user can participate in a cooperative environment with an expert user (e.g., proctor, instructor, or teacher) providing guidance. Systems and processes illustrated herein describe a cooperative environment where one or more remote users can view an expert user's movements and annotations. Such an expert-guided experience can improve education and reduce training time.

### Teleoperated Surgical System

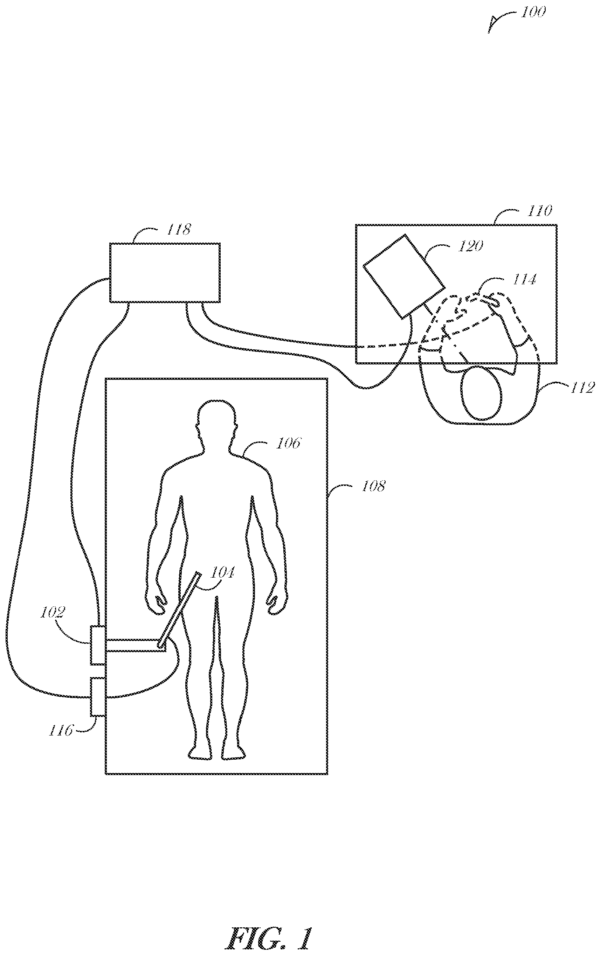

FIG. 1 is a schematic drawing illustrating a teleoperated surgical system 100, according to an embodiment. The teleoperated surgical system 100 includes a surgical manipulator assembly 102 for controlling operation of a surgical instrument 104 in performing various procedures on a patient 106. The assembly 102 is mounted to or located near an operating table 108. A user interface, such as master assembly 110, allows a surgeon 112 to view the surgical site and to control the manipulator assembly 102.

In alternative embodiments, the teleoperated surgical system 100 can include more than one manipulator assembly 102. The exact number of manipulator assemblies will depend on the surgical procedure and the space constraints within the operating room among other factors.

The master assembly 110 can be located in the same room as the operating table 108. However, it should be understood that the surgeon 112 can be located in a different room or a completely different building from the patient 106. The master assembly 110 generally includes one or more control device(s) 114 for controlling the manipulator assembly 102. The control device(s) 114 can include any number of a variety of input devices, such as gravity-balanced arms, joysticks, trackballs, gloves, trigger grips, hand-operated controllers, hand motion sensors, voice recognition devices, eye motion sensors, or the like. In some embodiments, the control device(s) 114 can be provided with the same degrees of freedom as the associated surgical instruments 104 to provide the surgeon 112 with telepresence, or the perception that the control device(s) 114 are integral with the instrument 104 so that the surgeon 112 has a strong sense of directly controlling the instrument 104. In some embodiments, the control device 114 is a manual input device that moves with six degrees of freedom or more, and which can also include an actuatable handle or other control feature (e.g., one or more buttons, switches, etc.) for actuating instruments (for example, for closing grasping jaws, applying an electrical potential to an electrode, delivering a medicinal treatment, or the like).

A visualization system 116 provides a concurrent two- or three-dimensional video image of a surgical site to surgeon 112. The visualization system 116 can include a viewing scope assembly. In some embodiments, visual images can be captured by an endoscope positioned within the surgical site. The visualization system 116 can be implemented as hardware, firmware, software, or a combination thereof, and it interacts with or is otherwise executed by one or more computer processors, which can include the one or more processors of a control system 118.

A display system 120 can display a visual image of the surgical site and surgical instruments 104 captured by the visualization system 116. The display system 120 and the master control devices 114 can be oriented such that the relative positions of the visual imaging device in the scope assembly and the surgical instruments 104 are similar to the relative positions of the surgeon's eyes and hands so the operator (e.g., surgeon 112) can manipulate the surgical instrument 104 with the master control devices 114 as if viewing a working volume adjacent to the instrument 104 in substantially true presence. By "true presence" it is meant that the presentation of an image is a true perspective image simulating the viewpoint of an operator that is physically manipulating the surgical instruments 104.

The control system 118 includes at least one processor (not shown) and typically a plurality of processors for effecting control between the surgical manipulator assembly 102, the master assembly 114, and the display system 116. The control system 118 also includes software programming instructions to implement some or all of the methods described herein. While control system 118 is shown as a single block in the simplified schematic of FIG. 1, the control system 118 can comprise a number of data processing circuits (e.g., on the surgical manipulator assembly 102 and/or on the master assembly 110). Any of a wide variety of centralized or distributed data processing architectures can be employed. Similarly, the programming code can be implemented as a number of separate programs or subroutines, or it can be integrated into a number of other aspects of the teleoperated systems described herein. In various embodiments, the control system 118 can support wireless communication protocols, such as Bluetooth, IrDA, HomeRF, IEEE 802.11, DECT, and Wireless Telemetry.

In some embodiments, the control system 118 can include servo controllers to provide force and torque feedback from the surgical instrument 104 to the master assembly 114. Any suitable conventional or specialized servo controller can be used. A servo controller can be separate from, or integral with, the manipulator assembly 102. In some embodiments, the servo controller and the manipulator assembly 102 are provided as part of a robotic arm cart positioned adjacent to the patient 106. The servo controllers transmit signals instructing the manipulator assembly 102 to move the instrument 104, which extends into an internal surgical site within the patient body via openings in the body.

Each manipulator assembly 102 supports at least one surgical instrument 104 (e.g., "slave") and can comprise a series of non-teleoperated, manually articulatable linkages and a teleoperated robotic manipulator. The linkages can be referred to as a set-up structure, which includes one or more links coupled with joints that allows the set-up structure to be positioned and held at a position and orientation in space. The manipulator assembly 102 can be driven by a series of actuators (e.g., motors). These motors actively move the robotic manipulators in response to commands from the control system 118. The motors are further coupled to the surgical instrument 104 so as to advance the surgical instrument 104 into a naturally or surgically created anatomical orifice and move the surgical instrument 104 in multiple degrees of freedom that can include three degrees of linear motion (e.g., X, Y, Z linear motion) and three degrees of rotational motion (e.g., roll, pitch, yaw). Additionally, the motors can be used to actuate an effector of the surgical instrument 104 such as an articulatable effector for grasping tissues in the jaws of a biopsy device or an effector for obtaining a tissue sample or for dispensing medicine, or another effector for providing other treatment as described more fully below. For example, the instrument 104 can be pitched and yawed around the remote center of motion, and it can be inserted and withdrawn through the remote center of motion (e.g., the z-axis motion). Other degrees of freedom can be provided by moving only part of the instrument (e.g., the end effector). For example, the end effector can be rolled by rolling the shaft, and the end effector is pitched and yawed at a distal-end wrist.

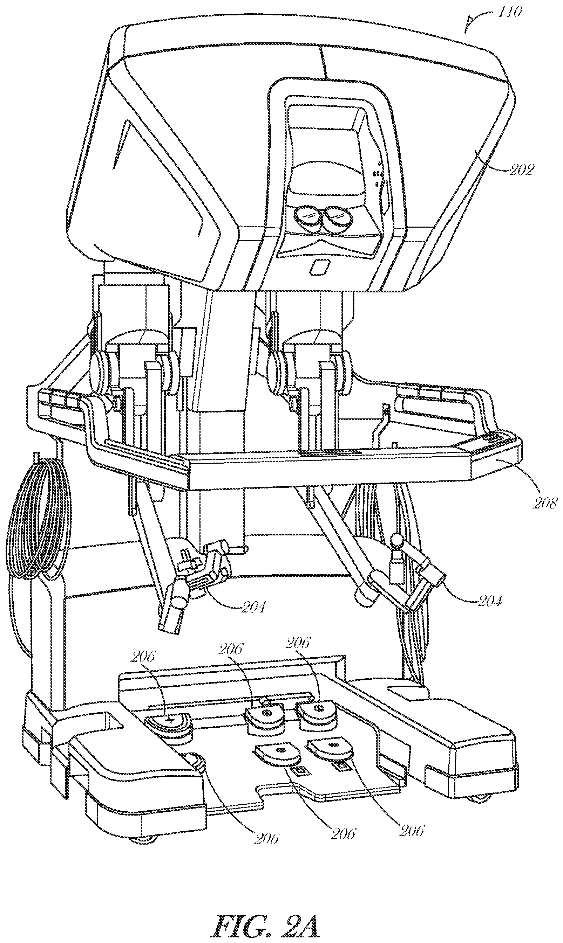

FIG. 2A is a drawing illustrating a master assembly 110, an example of a user interface usable by a user to control manipulator assembly 102 (shown at FIG. 1). A user may sit at the master assembly 110 and may access a display system 202, master controllers 204, and footswitch panel 206. The footswitch panel 206 enables the user to perform various tasks, such as swapping between various surgical instruments or controlling video or camera features. While seated at the master assembly 110, the user may rest their arms on an armrest 208. When operating in a live surgery, the display system 202 displays the surgical field as captured from a camera inserted through a small opening to the surgical site, sometimes referred to as a portal or a cannula. A user interface such as master assembly 110, without one or more corresponding manipulator assemblies (e.g., manipulator assembly 102 shown at FIG. 1), can also be used to train users on the use of a teleoperated surgical system (e.g., teleoperated surgical system 100 shown at FIG. 1). For training purposes, a simulated environment may be displayed on the display system 202, where the simulated environment may be a stereoscopic display of a surgical site and virtual slave surgical instruments. As the user moves the master controllers 204, a virtual surgical instrument may move in a corresponding fashion in the stereoscopic display.

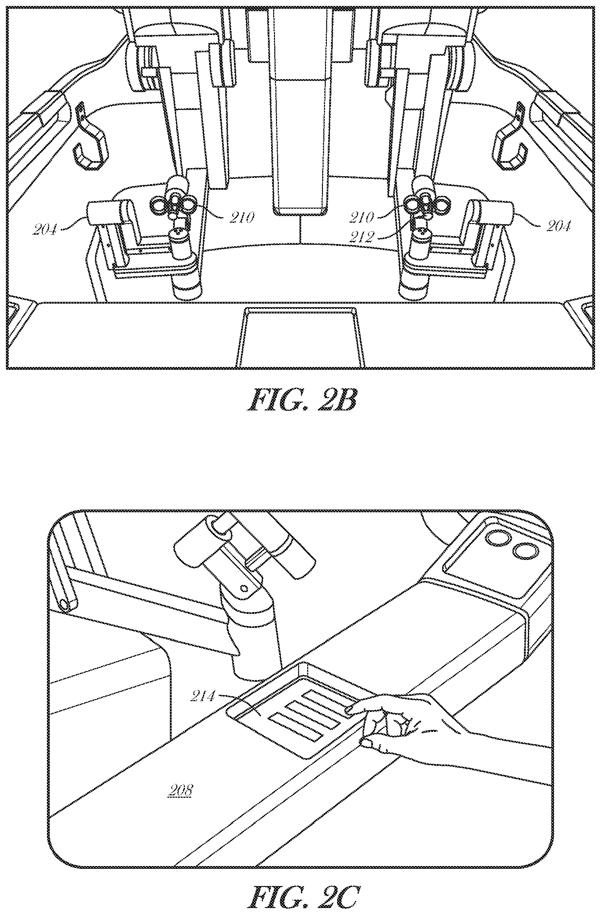

FIG. 2B is a drawing illustrating a master controller 204 of a master assembly 110, according to an embodiment. The master controller 204 includes a handheld part or gimbal. The master controller 204 has an articulated arm portion including a plurality of members or links connected together by pivotal connections or joints. The user grips finger loops 210 by positioning his or her thumb and index finger over a pincher formation 212. The user's thumb and index finger are typically held on the pincher formation by straps threaded through slots to create the finger loops 210. When the pincher formation 212 is squeezed between the thumb and index finger, the fingers or other element of the surgical instrument 104 move in synchronicity. The joints of the master controller 204 are operatively connected to actuators, e.g., electric motors, or the like, to provide for, e.g., force feedback, gravity compensation, and the like. Furthermore, appropriately positioned sensors, e.g., encoders, or potentiometers, or the like, are positioned on each joint of the master controller 204, so as to enable joint positions of the master controller 204 to be determined by the master assembly 110 or other control systems in the teleoperated surgical system 100.

In an embodiment, there are two master controllers 204, each with two finger loops 210 for which the user may insert an index finger and thumb of a respective hand. The two master controllers 204 may each control a virtual surgical instrument. The user may be provided software or hardware mechanisms to swap between multiple instruments for one or both master controller 204. For example, a user may be provided three instruments, such as two forceps and a retractor. One or both of the forceps may be an energy instrument able to cauterize tissue. The user may first use the forceps at each master controller 204, then switch the right master controller 204 to control the retractor to expose a section of the surgical field, and then switch the right master controller 204 back to the forceps to continue cutting, probing, or dissecting tissue.

While using the master controllers 204, the user is provided with full three-dimensional range of motion (x, y, and z axis) along with rotational motion (roll, pitch, yaw) in addition to pinching motion with the index and thumb (or any two fingers inserted into the loops 210). As such, by moving the appropriate master controller 204, the user is able to manipulate the corresponding surgical instrument through a full range of motion.

FIG. 2C is a drawing illustrating an armrest 208 of a master assembly 110, according to an embodiment. The armrest 208 may include one more touch controls, such as touchscreens, soft buttons, mechanical buttons, or the like. In the example illustrated in FIG. 2C, a single touchscreen 214 is shown through which the user may configure various video, audio, or other system settings.

In an embodiment, the display system 120 can display a virtual environment simulating a surgical site within a patient. The virtual environment can include various biological structures in addition to the surgical instrument 104. The surgeon 112 operates the instrument 104 within the virtual environment to train, obtain certification, or experiment with various skills or procedures without having the possibility of harming a real patient. Simulating surgical procedures also has the advantage of requiring fewer components. For example, a patient-side cart is not needed because there is no actual patient. Thus, simulation provides increased convenience and accessibility.

### Overview of Virtual Training Environment

Disclosed herein is a virtual training environment that includes a local user's virtual surgical instruments rendered in a virtual surgical environment along with an expert user's surgical instruments. One goal is to obtain more consistent training outcomes. Another goal is to reduce training time. Yet other goals include, but are not limited to, providing a more engaging and interactive training environment and providing a platform for expert feedback to increase training efficacy.

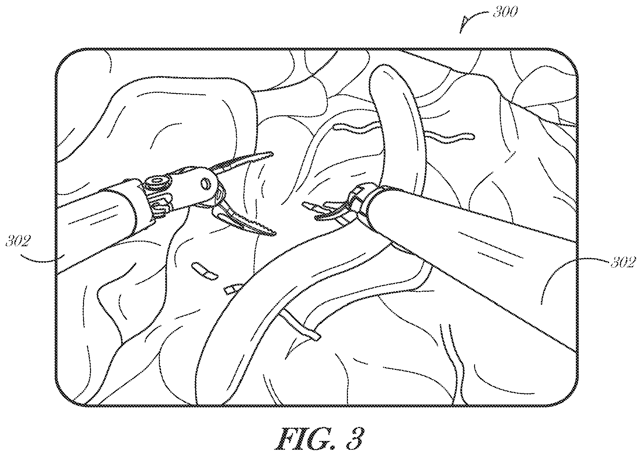

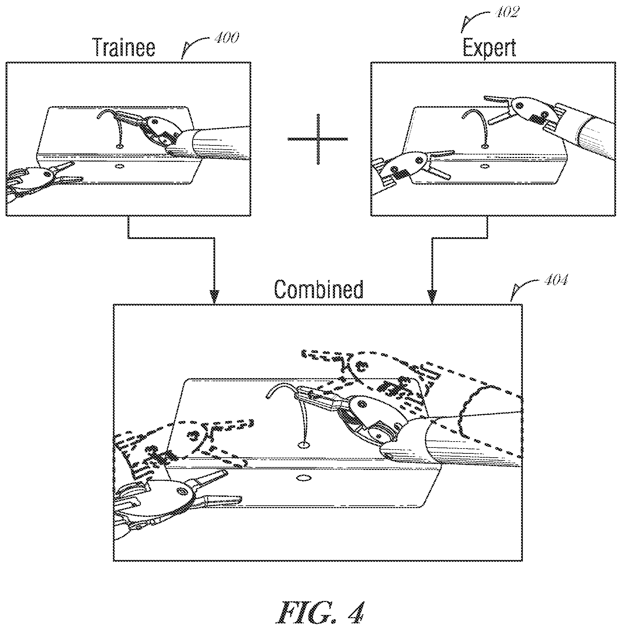

FIG. 3 illustrates a virtual surgical site 300, according to an embodiment. The virtual surgical site 300 may be displayed on the display system 202 and includes two virtual slave surgical instruments 302. In a cooperative training environment, a second set of virtual surgical instruments can be overlaid on the user's display. The second set of virtual surgical instruments can be representations of virtual instruments controlled by an expert user (e.g., proctor, instructor, teacher, etc.). FIG. 4 illustrates a process to composite two virtual surgical sites, according to an embodiment. The trainee can operate in one virtual environment, which can be rendered in a trainee scene 400. Similarly, the expert user can view the same or similar environment and have control of separate virtual surgical instruments. The expert scene 402 is rendered separately. The combined scene 404 is the composite of the trainee scene 400 and the expert scene 402 and is output to the trainee at the master assembly. Similarly, a combined scene is output to the expert's master assembly.

The expert user's surgical instruments can be presented in a translucent or semi-transparent overlay in the trainee's screen (represented by the dashed outline virtual instruments in the combined scene 404). In this manner, the expert user who is operating a separate master assembly is able to visually guide or advise the trainee user and the trainee can mimic or watch the expert's virtual instruments in the display system. Other visual effects can be applied to the expert user's surgical instruments, such as a semi-transparent effect, see-through effect, or an abstracted representation (e.g., a dotted outline, ghosted shape, cartoon drawing, etc.). Optionally, in some embodiments, the expert user's surgical instruments are rendered in a manner to resemble the trainee user's virtual surgical instruments (e.g., opaque, shaded, etc.). Further, while some embodiments are described with the expert's virtual surgical instruments being visually modified (e.g., using a semi-transparent effect), it is understood that such modifications can be applied to the trainee user's virtual instruments. For example, in an embodiment, at the expert user's station, the expert user's virtual instruments are rendered as opaque while the trainee's virtual instruments are rendered as semi-transparent or see-through. Additionally, the effect used on the virtual instrument (either trainee or expert) can be modified before or during an exercise. The modifications can be used to improve training methodologies.

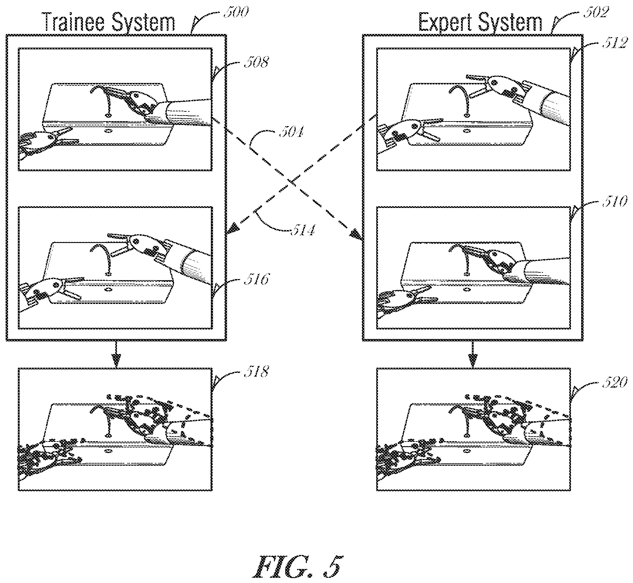

FIG. 5 is a data flow diagram illustrating cooperative data sharing between a trainee system 500 and a proctor system 502, according to an embodiment. In one embodiment, each of the trainee system 500 and the proctor system 502 is a teleoperated surgical system (e.g., teleoperated surgical system 100 shown at FIG. 1). In an alternate embodiment, at least one of the trainee system 500 and the proctor system 502 comprises a user interface component of a teleoperated surgical system (e.g., master assembly 110 shown at FIG. 2A) without one or more associated manipulator assemblies (e.g., manipulator assembly 102 shown at FIG. 1). When the user (e.g., trainee) at the trainee system operates the master assembly via the master control devices (e.g., master controllers, foot pedals, etc.), the trainee system 500 receives input data, such as the position, speed, or state of the various master control devices. Some or all of the input data received at the trainee system is transmitted to the expert system (arrow 504). The input data is used to render the position and state of the virtual surgical instruments on the trainee system 500 as a local scene 508. Similarly, the input data is used on the expert system 502 to render the environment of the trainee system 510. This is a remote scene from the perspective of the user at the expert system 502.

In a similar fashion, some or all of the input data received at the expert system 502 as a result of a user's operation of the expert system 502 is transmitted to the trainee system 500. At the expert system 502, the input data received at the expert system 502 as a result of the user's operation of the expert system 502 is used to render a local scene 512 (local with respect to the user at the expert system 502). The input data received at the expert system 502 as a results of the user's operation of the expert system 502 is transmitted (arrow 514) to the trainee system 500 and rendered as a remote scene 516 (remote with respect to the trainee system 500).

The trainee system 500 renders a composite scene 518 that includes the local scene 508 and the remote scene 516. The composite scene 518 may alter the remote scene 516 using various graphical manipulations, for example making the remote scene 516 translucent, changing the color of the remote virtual instruments, or other enhancements to allow the user of the trainee system 500 to more easily distinguish the local virtual surgical instruments from the remote (e.g., expert) surgical instruments in the composite scene 518. The expert system 502 produces a similar composite scene 520 to provide the expert system 502 user a view of the local and remote virtual surgical instruments. The expert system 502 can optionally alter the local scene 512 or the remote scene 510 (local and remote from the perspective of the expert system 502) using various graphical manipulations, for example by making the local scene 512 or remote scene 510 translucent, semi-transparent, changing the color of the virtual instruments, etc.

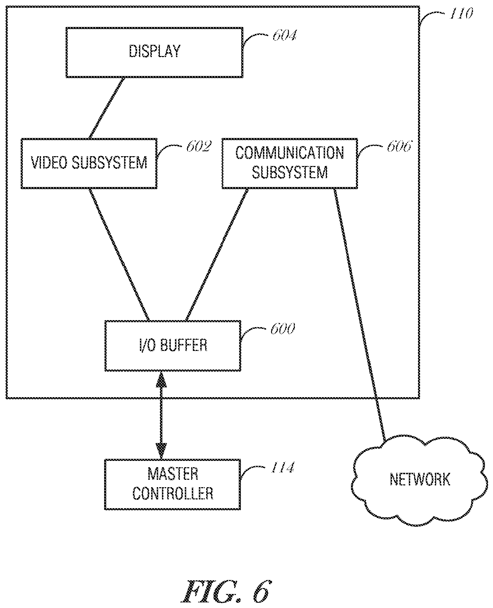

FIG. 6 is a block diagram illustrating a master assembly 110. Master assembly 110 is one embodiment of a user interface that can be used to control, in a teleoperated surgical system, one or more surgical instruments (e.g., surgical instrument 104 shown at FIG. 1) through associated manipulator assemblies (e.g., manipulator assembly 102 at FIG. 1). Master assembly 110 can also be used to perform simulated procedures in virtual environments, to train persons in the use of a teleoperated surgical system. As the user manipulates the master controller 114 to control virtual surgical instruments in a virtual surgical simulation, input signals are transmitted to an input/output (I/O) buffer 600. Input signals include various arm movements and positions (e.g., of master controller 204), camera controls, or other inputs received from a user at the master assembly 110. The input control signals can be scanned, filtered, and processed to identify input control signals that affect the virtual surgical simulation. Such input control signals are sent to a video subsystem 602 at the master assembly 110. The video subsystem 602 can include video processors, video memory, and other components to render a video image for presentation on a display 604. The input control signals are also sent to a communication module 606. The communication subsystem 606 transmits the input control signals to another (remote) master assembly 110 (not shown), which can then use the input control signals as if they were generated local to the (remote) master assembly 110. The communication subsystem 606 is also able to receive input control signals from the remote master assembly 110, where the received input control signals are representative of actions taken by a remote user of the remote master assembly 110. Input control signals received from a remote user are forwarded to the I/O buffer 600, which then communicates them to the video subsystem 602 for processing.

It is understood that more than one remote master assembly 110 can receive the input control signals from the communication subsystem 606 and that the communication subsystem 606 can receive input control signals from more than one remote master assembly 110. In this manner, several instructors may provide concurrent instruction or guidance to a local user, each instructor having virtual surgical instruments represented in the local user's display. Also in this manner, several trainee users may receive instruction from one or more instructors. While FIG. 6 illustrates that the communication subsystem 606 receives the input control signals from the I/O buffer 600, it is understood that the communication subsystem 606 can receive input control signals from other intermediate sources, such as an operating system, a device driver, an application, or other middleware.

The communication subsystem 606 can communicate with the remote master assembly 110 using various networking protocols or technologies, such as a local area network (LAN), a wide area network (WAN), the Internet, mobile telephone networks, Plain Old Telephone (POTS) networks, and wireless data networks (e.g., Wi-Fi, 3G, and 4G LTE/LTE-A or WiMAX networks).

FIG. 6 illustrates a system for managing a user interface that includes a first user interface (e.g., master assembly 110) with a communications subsystem 606 configured to receive at the first user interface from a second user interface, an environmental variable describing operation of the second user interface. The first user interface also includes a video subsystem 602 to render a local scene at the first user interface, the local scene representing a state of operation of the first user interface. The video subsystem 602 renders a remote scene at the first user interface, the remote scene representing a state of operation of the second user interface and the remote scene based at least in part on the environmental variable. Then, the video subsystem 602 composites the local scene and the remote scene to produce a composite scene and presents the composite scene to a user of the first user interface via the display 604.

The environmental variable can be represented as a data structure of one or more n-tuples. For example, the *n*-tuple can be a 4-tuple as (*input_id, x-position, y-position, z-position*). In some embodiments, the *input_id* is used to uniquely identify an input of a user interface of a teleoperated surgical system. For example, the value "1" can correspond to a left master controller and the value "2" can correspond to a right master controller. As such, the 4-tuple of (1, 33.4, 24.9, 18.4) represents that the position of the left master controller is 33.4 cm in the *x-position,* 24.9 cm in the *y-position,* and 18.4 cm in the *z-position.* The master assembly can translate the x-y-z position into a corresponding position in the virtual environment to correctly represent the position, attitude, or speed of a virtual surgical instrument corresponding to the left master controller. The same 4-tuple can be used locally to render a local scene or transmitted to a remote master controller of a teleoperated surgical system to render a scene. Transmitting the n-tuple is advantageous in that it reduces network load and decreases latency.

In another embodiment, the pose of the master controller in addition to its x-y-z position is transmitted from one master assembly to another. This gives the orientation of the wrist. The value of open/close of the instrument pincher formation is also transmitted. A 4x4 transform matrix with a 3x3 rotation matrix in the upper left and a 3x1 translation vector in the upper right is used. In addition, the *input-id* indicates left/right hand, which remote user it is (in the case where there are multiple remote users), and the open/close position of the grippers (between 0 and 1, with 0 being fully open and 1 being fully closed) are transmitted.

In an embodiment, the second user interface includes a master controller operated by a user of the second user interface, and the environmental variable includes a position, speed, or rotation of the master controller.

In an embodiment, the communication subsystem 606 is further configured to receive an annotation variable from the second user interface, the annotation variable describing an annotation to render on the composite scene. In such an embodiment, the video subsystem 602 is further configured to composite the composite scene to include the annotation and present the annotation in the composite scene. In an embodiment, the annotation includes a crayon control, a high lighter control, or a pointer icon. For example, the remote user (e.g., proctor, instructor, teacher, etc.) can use a master controller 204 to control a crayon icon to draw arrows, circles, dashes, etc. on the shared screens in order to annotate them. Annotations can be provided as text, figures (e.g., circles, squares, etc.), free-form drawing, pictures, icons, or the like. The annotation can be selected by a user of the second user interface.

Annotations can be rendered in the world coordinate frame so that they are tied to the environment and not to a particular camera reference frame. In this configuration, annotations are able to persist at a given location in the environment regardless of changes in camera angle. For example, an expert can annotate a dot on a suture sponge that the trainee is to focus on during practice, where the dot maintains a persistent location on the sponge during the exercise regardless of camera changes.

In an embodiment, the environmental variable includes a camera control variable. In such an embodiment, the video subsystem 602 is further configured to render the local scene includes rendering the local scene using the camera control variable.

In an embodiment, the local scene includes a virtual surgical instrument controlled by the user of the first user interface.

In an embodiment, the video subsystem is further configured to render the remote scene as a translucent layer, the translucent layer allowing the user of the first user interface to view the local scene when viewing the composite scene.

In an embodiment, the master assembly can include a training subsystem to provide a surgical exercise to the user of the first user interface, where the surgical exercise is also substantially concurrently provided to a user of the second user interface.

In an embodiment, the communication subsystem 606 is configured to receive the environmental variable over a wide-area network. In an embodiment, the wide-area network comprises the Internet. In an embodiment, the wide-area network comprises a wireless network.

In an embodiment, the video subsystem 602 is configured to render the local scene and the remote scene on separate canvases.

In an embodiment, the video subsystem 602 is configured to render the composite scene on a separate canvas from the rendering the local scene.

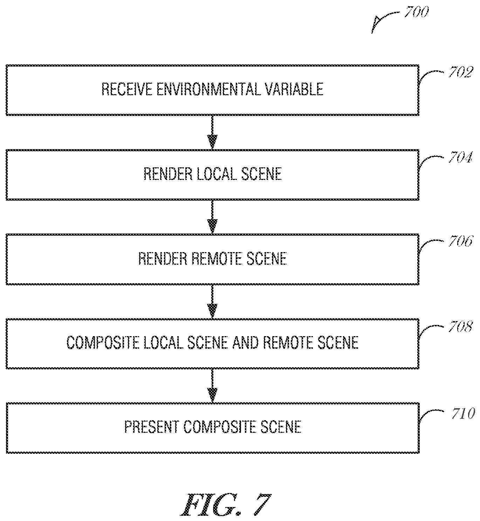

FIG. 7 is a flowchart illustrating a method 700 of scoring a teleoperated surgical training session, according to an embodiment. At block 702, an environmental variable describing operation of a second user interface is received at a first user interface from a second user interface. In an embodiment, the second user interface includes a master controller operated by a user of the second user interface, and wherein the environmental variable includes a position, speed, or rotation of the master controller. In an embodiment, the environmental variable includes a camera control variable, and wherein rendering the local scene includes rendering the local scene using the camera control variable.

In an embodiment, receiving the environmental variable comprises receiving the environmental variable over a wide-area network. In an embodiment, the wide-area network comprises the Internet. In an embodiment, the wide-area network comprises a wireless network.

At block 704, a local scene is rendered at the first user interface, the local scene representing a state of operation of the first user interface.

At block 706, a remote scene is rendered at the first user interface, the remote scene representing a state of operation of the second user interface and the remote scene based at least in part on the environmental variable. In an embodiment, rendering the remote scene comprises rendering the remote scene as a translucent layer, the translucent layer allowing the user of the first user interface to view the local scene when viewing the composite scene.

At block 708, the local scene and the remote scene is composited to produce a composite scene. In an embodiment, the local scene includes a virtual surgical instrument controlled by the user of the first user interface. In an embodiment, rendering the local scene and rendering the remote scene are performed on separate canvases.

At block 710, the composite scene is presented to a user of the first user interface. In an embodiment, rendering the composite scene is performed on a separate canvas from the rendering the local scene.

In an embodiment, the method 700 includes receiving an annotation variable from the second user interface, the annotation variable describing an annotation to render on the composite scene; and compositing the composite scene to include the annotation; where presenting the composite scene includes presenting the annotation in the composite scene. In an embodiment, the annotation includes a crayon control, a high lighter control, or a pointer icon. In an embodiment, the annotation is selected by a user of the second user interface.

In a further embodiment, the method 700 includes providing a surgical exercise to the user of the first user interface, and wherein the surgical exercise is also substantially concurrently provided to a user of the second user interface.

### Computer Hardware and Storage Devices

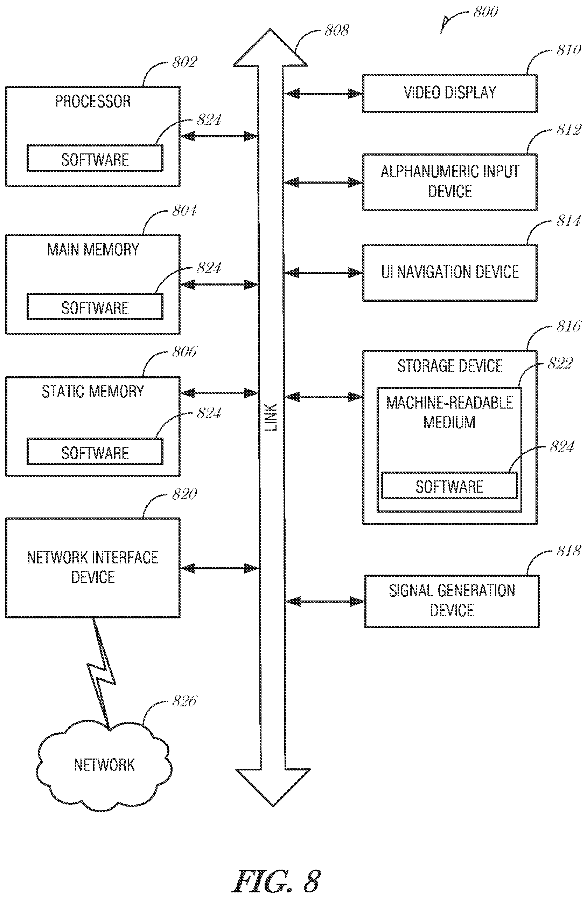

FIG. 8 is a block diagram illustrating a machine in the example form of a computer system 800, within which a set or sequence of instructions for causing the machine to perform any one of the methodologies discussed herein may be executed, according to an example embodiment. In alternative embodiments, the machine operates as a standalone device or may be connected (e.g., networked) to other machines. In a networked deployment, the machine may operate in the capacity of either a server or a client machine in server-client network environments, or it may act as a peer machine in peer-to-peer (or distributed) network environments. The machine may be a personal computer (PC), a tablet PC, a set-top box (STB), a Personal Digital Assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein.

Example computer system 800 includes at least one processor 802 (e.g., a central processing unit (CPU), a graphics processing unit (GPU) or both, processor cores, compute nodes, etc.), a main memory 804 and a static memory 806, which communicate with each other via a link 808 (e.g., bus). The computer system 800 may further include a video display unit 810, an alphanumeric input device 812 (e.g., a keyboard), and a user interface (UI) navigation device 814 (e.g., a mouse). In one embodiment, the video display unit 810, input device 812 and UI navigation device 814 are incorporated into a touch screen display. The computer system 800 may additionally include a storage device 816 (e.g., a drive unit), a signal generation device 818 (e.g., a speaker), a network interface device 820, and one or more sensors (not shown), such as a global positioning system (GPS) sensor, compass, accelerometer, or other sensor.

The storage device 816 includes a machine-readable medium 822 on which is stored one or more sets of data structures and instructions 824 (e.g., software) embodying or utilized by any one or more of the methodologies or functions described herein. The instructions 824 may also reside, completely or at least partially, within the main memory 804, static memory 806, and/or within the processor 802 during execution thereof by the computer system 800, with the main memory 804, static memory 806, and the processor 802 also constituting machine-readable media.

While the machine-readable medium 822 is illustrated in an example embodiment to be a single medium, the term "machine-readable medium" may include a single medium or multiple media (e.g., a centralized or distributed database, and/or associated caches and servers) that store the one or more instructions 824. The term "machine-readable medium" shall also be taken to include any tangible medium that is capable of storing, encoding or carrying instructions for execution by the machine and that cause the machine to perform any one or more of the methodologies of the present disclosure or that is capable of storing, encoding or carrying data structures utilized by or associated with such instructions. The term "machine-readable medium" shall accordingly be taken to include, but not be limited to, solid-state memories, and optical and magnetic media. Specific examples of machine-readable media include non-volatile memory, including, by way of example, semiconductor memory devices (e.g., Electrically Programmable Read-Only Memory (EPROM), Electrically Erasable Programmable Read-Only Memory (EEPROM)) and flash memory devices; magnetic disks such as internal hard disks and removable disks; magneto-optical disks; and CD-ROM and DVD-ROM disks.

The instructions 824 may further be transmitted or received over a communications network 826 using a transmission medium via the network interface device 820 utilizing any one of a number of well-known transfer protocols (e.g., HTTP). Examples of communication networks include a local area network (LAN), a wide area network (WAN), the Internet, mobile telephone networks, Plain Old Telephone (POTS) networks, and wireless data networks (e.g., Wi-Fi, 3G, and 4G LTE/LTE-A or WiMAX networks). The term "transmission medium" shall be taken to include any intangible medium that is capable of storing, encoding, or carrying instructions for execution by the machine, and includes digital or analog communications signals or other intangible medium to facilitate communication of such software.

It will be appreciated that, for clarity purposes, the above description describes some embodiments with reference to different functional units or processors. However, it will be apparent that any suitable distribution of functionality between different functional units, processors or domains can be used without detracting from the present disclosure. For example, functionality illustrated to be performed by separate processors or controllers can be performed by the same processor or controller. Hence, references to specific functional units are only to be seen as references to suitable means for providing the described functionality, rather than indicative of a strict logical or physical structure or organization.

Although the present disclosure has been described in connection with some embodiments, it is not intended to be limited to the specific form set forth herein. One skilled in the art would recognize that various features of the described embodiments can be combined in accordance with the present disclosure. Moreover, it will be appreciated that various modifications and alterations can be made by those skilled in the art without departing from the scope of the present disclosure.

In addition, in the foregoing detailed description, it can be seen that various features are grouped together in a single embodiment for the purpose of streamlining the disclosure. This method of disclosure is not to be interpreted as reflecting an intention that the claimed embodiments require more features than are expressly recited in each claim. Rather, as the following claims reflect, inventive subject matter lies in less than all features of a single disclosed embodiment. Thus the following claims are hereby incorporated into the detailed description, with each claim standing on its own as a separate embodiment.

The foregoing description and drawings of embodiments in accordance with the present invention are merely illustrative of the principles of the inventive subject matter. Therefore, it will be understood that various modifications can be made to the embodiments by those skilled in the art without departing from the scope of the inventive subject matter, which is defined in the appended claims.

Thus, while certain exemplary embodiments of the invention have been described and shown in the accompanying drawings, it is to be understood that such embodiments are merely illustrative of and not restrictive on the broad inventive subject matter, and that the embodiments of the invention not be limited to the specific constructions and arrangements shown and described, since various other modifications can occur to those ordinarily skilled in the art.

## Claims

1. A method of managing a user interface of a teleoperated surgical system, the method comprising:
receiving (702) at a first user interface (110) an environmental variable, transmitted from a second user interface and describing operation of a remote virtual instrument in the second user interface, the environmental variable being a data structure of one or more n-tuples, wherein the remote virtual instrument is operated by a user of the second user interface and the environmental variable includes a camera control variable;
rendering (704) a local scene at the first user interface (110) using the camera control variable, the local scene representing a state of operation of the first user interface, the local scene representing a virtual surgical site including a local virtual instrument, wherein the local virtual instrument is controlled by the user of the first user interface;
rendering (706) a remote scene at the first user interface (110), the remote scene representing a state of operation of the second user interface, the remote scene representing the virtual surgical site including the remote virtual instrument, wherein the remote scene is generated based at least in part on the environmental variable;
compositing (708) the local scene and the remote scene to produce a virtual composite scene; and
presenting (710) the virtual composite scene to the user of the first user interface (110), wherein the virtual composite scene includes the local scene and the remote scene, wherein the remote scene is altered using graphical manipulations to distinguish the local virtual instrument from the remote virtual instrument.

2. The method of claim 1, wherein the second user interface includes a master controller operated by the user of the second user interface, and wherein the environmental variable includes a position, speed, or rotation of the operation of the master controller.

3. The method of claim 1, further comprising:
receiving an annotation variable from the second user interface, the annotation variable describing an annotation to render on the virtual composite scene; and
compositing the virtual composite scene to include the annotation;
wherein presenting the virtual composite scene includes presenting the annotation in the virtual composite scene.

4. The method of claim 3, wherein the annotation includes a crayon control, a highlighter control, or a pointer icon.

5. The method of claim 4, wherein the annotation is selected by a user of the second user interface.

6. The method of claim 1, wherein the local virtual instrument is a virtual surgical instrument.

7. The method of claim 1, wherein rendering the remote scene comprises rendering the remote scene as a translucent layer, the translucent layer allowing the user of the first user interface to view the local scene when viewing the virtual composite scene.

8. The method of claim 1, further comprising:
providing a surgical exercise to the user of the first user interface (110), and wherein the surgical exercise is also substantially concurrently provided to a user of the second user interface.

9. The method of claim 1, wherein rendering the local scene and rendering the remote scene are performed on separate canvases.

10. A system for managing a user interface of a teleoperated surgical system, the system comprising:
a first user interface (110) comprising:
a communications subsystem (606) configured to receive at the first user interface from a second user interface, an environmental variable describing one or more operations of a remote virtual instrument in the second user interface, the environmental variable being a data structure of one or more n-tuples and wherein the environmental variable includes a camera control variable; and
a video subsystem (602) configured to:
render (704) a local scene at the first user interface (110) using the camera control variable, the local scene representing a state of operation of the first user interface, the local scene representing a virtual surgical site including a local virtual instrument, and wherein the local virtual instrument is controlled by the user of the first user interface;
render (706) a remote scene at the first user interface (110), the remote scene representing a state of operation of the second user interface, the remote scene representing the virtual surgical site including the remote virtual instrument,
wherein the remote scene is generated based at least in part on the environmental variable;
composite (708) the local scene and the remote scene to produce a virtual composite scene; and
present (710) the virtual composite scene to a user of the first user interface (110), wherein the virtual composite scene includes the local scene and the remote scene, wherein the remote scene is altered using graphical manipulations to distinguish the local virtual instrument from the remote virtual instrument.

11. The system of claim 10, wherein the second user interface includes a master controller operated by the user of the second user interface, and wherein the environmental variable includes a position, speed, or rotation of the operation of the master controller.

12. The system of claim 10, wherein the communications subsystem (606) is further configured to receive an annotation variable from the second user interface, the annotation variable describing an annotation to render on the virtual composite scene; and
wherein the video subsystem (602) is further configured to:
composite the virtual composite scene to include the annotation; and
present the annotation in the virtual composite scene.

13. The system of claim 10, wherein the video subsystem (602) is further configured to render the remote scene as a translucent layer, the translucent layer allowing the user of the first user interface to view the local scene when viewing the virtual composite scene.

## Patentansprüche

1. Ein Verfahren zum Verwalten einer Benutzeroberfläche eines teleoperierten chirurgischen Systems, wobei das Verfahren Folgendes umfasst:
Empfangen (702) an einer ersten Benutzeroberfläche (110) einer Umgebungsvariablen, die von einer zweiten Benutzeroberfläche übertragen wird und eine Operation eines entfernten virtuellen Instruments in der zweiten Benutzeroberfläche beschreibt, wobei die Umgebungsvariable eine Datenstruktur von einem oder mehreren n-Tupeln ist, wobei das entfernte virtuelle Instrument von einem Benutzer der zweiten Benutzeroberfläche operiert wird und die Umgebungsvariable eine Kamerasteuerungsvariable beinhaltet;
Rendern (704) einer lokalen Szene an der ersten Benutzeroberfläche (110) unter Verwendung der Kamerasteuerungsvariablen, wobei die lokale Szene einen Operationszustand der ersten Benutzeroberfläche repräsentiert, wobei die lokale Szene einen virtuellen chirurgischen Ort, der ein lokales virtuelles Instrument beinhaltet, repräsentiert, wobei das lokale virtuelle Instrument von dem Benutzer der ersten Benutzeroberfläche gesteuert wird;
Rendern (706) einer entfernten Szene an der ersten Benutzeroberfläche (110), wobei die entfernte Szene einen Operationszustand der zweiten Benutzeroberfläche repräsentiert, wobei die entfernte Szene den virtuellen chirurgischen Ort, der das entfernte virtuelle Instrument beinhaltet, repräsentiert, wobei die entfernte Szene mindestens zum Teil basierend auf der Umgebungsvariablen generiert wird;
Zusammensetzen (708) der lokalen Szene und der entfernten Szene, um eine virtuelle zusammengesetzte Szene zu produzieren; und
Präsentieren (710) der virtuellen zusammengesetzten Szene für den Benutzer der ersten Benutzeroberfläche (110), wobei die virtuelle zusammengesetzte Szene die lokale Szene und die entfernte Szene beinhaltet, wobei die entfernte Szene unter Verwendung grafischer Manipulationen verändert wird, um das lokale virtuelle Instrument von dem entfernten virtuellen Instrument zu unterscheiden.

2. Verfahren nach Anspruch 1, wobei die zweite Benutzeroberfläche einen Mastercontroller beinhaltet, der von dem Benutzer der zweiten Benutzeroberfläche operiert wird, und wobei die Umgebungsvariable eine Position, Geschwindigkeit oder Drehung der Operation des Mastercontrollers beinhaltet.

3. Verfahren nach Anspruch 1, das ferner Folgendes umfasst:
Empfangen einer Annotationsvariablen von der zweiten Benutzeroberfläche, wobei die Annotationsvariable eine Annotation zum Rendern auf der virtuellen zusammengesetzten Szene beschreibt; und
Zusammensetzen der virtuellen zusammengesetzten Szene, um die Annotation zu beinhalten;
wobei das Präsentieren der virtuellen zusammengesetzten Szene das Präsentieren der Annotation in der virtuellen zusammengesetzten Szene beinhaltet.

4. Verfahren nach Anspruch 3, wobei die Annotation eine Zeichenstiftsteuerung, eine Leuchtstiftsteuerung oder ein Zeigersymbol beinhaltet.

5. Verfahren nach Anspruch 4, wobei die Annotation von einem Benutzer der zweiten Benutzeroberfläche selektiert wird.

6. Verfahren nach Anspruch 1, wobei das lokale virtuelle Instrument ein virtuelles chirurgisches Instrument ist.

7. Verfahren nach Anspruch 1, wobei das Rendern der entfernten Szene das Rendern der entfernten Szene als transluzente Schicht umfasst, wobei die transluzente Schicht dem Benutzer der ersten Benutzeroberfläche erlaubt, die lokale Szene zu betrachten, wenn er die virtuelle zusammengesetzte Szene betrachtet.

8. Verfahren nach Anspruch 1, das ferner Folgendes umfasst:
Bereitstellen einer chirurgischen Aufgabe für den Benutzer der ersten Benutzeroberfläche (110), und wobei die chirurgische Aufgabe im Wesentlichen auch zeitgleich für einen Benutzer der zweiten Benutzeroberfläche bereitgestellt wird.

9. Verfahren nach Anspruch 1, wobei das Rendern der lokalen Szene und das Rendern der entfernten Szene auf getrennten Zeichenoberflächen durchgeführt werden.

10. Ein System zum Verwalten einer Benutzeroberfläche eines teleoperierten chirurgischen Systems, wobei das System Folgendes umfasst:
eine erste Benutzeroberfläche (110), die Folgendes umfasst:
ein Kommunikationsteilsystem (606), das dazu konfiguriert ist, an der ersten Benutzeroberfläche von einer zweiten Benutzeroberfläche eine Umgebungsvariable zu empfangen, die eine oder mehrere Operationen eines entfernten virtuellen Instruments in der zweiten Benutzeroberfläche beschreibt, wobei die Umgebungsvariable eine Datenstruktur von einem oder mehreren n-Tupeln ist und wobei die Umgebungsvariable eine Kamerasteuerungsvariable beinhaltet; und
ein Videoteilsystem (602), das zu Folgendem konfiguriert ist:
Rendern (704) einer lokalen Szene an der ersten Benutzeroberfläche (110) unter Verwendung der Kamerasteuerungsvariablen, wobei die lokale Szene einen Operationszustand der ersten Benutzeroberfläche repräsentiert, wobei die lokale Szene einen virtuellen chirurgischen Ort, der ein lokales virtuelles Instrument beinhaltet, repräsentiert, und wobei das lokale virtuelle Instrument von dem Benutzer der ersten Benutzeroberfläche gesteuert wird;
Rendern (706) einer entfernten Szene an der ersten Benutzeroberfläche (110), wobei die entfernte Szene einen Operationszustand der zweiten Benutzeroberfläche repräsentiert, wobei die entfernte Szene den virtuellen chirurgischen Ort, der das entfernte virtuelle Instrument beinhaltet, repräsentiert, wobei die entfernte Szene mindestens zum Teil basierend auf der Umgebungsvariablen generiert wird;
Zusammensetzen (708) der lokalen Szene und der entfernten Szene, um eine virtuelle zusammengesetzte Szene zu produzieren; und
Präsentieren (710) der virtuellen zusammengesetzten Szene für einen Benutzer der ersten Benutzeroberfläche (110), wobei die virtuelle zusammengesetzte Szene die lokale Szene und die entfernte Szene beinhaltet, wobei die entfernte Szene unter Verwendung grafischer Manipulationen verändert wird, um das lokale virtuelle Instrument von dem entfernten virtuellen Instrument zu unterscheiden.

11. System nach Anspruch 10, wobei die zweite Benutzeroberfläche einen Mastercontroller beinhaltet, der von dem Benutzer der zweiten Benutzeroberfläche operiert wird, und wobei die Umgebungsvariable eine Position, Geschwindigkeit oder Drehung der Operation des Mastercontrollers beinhaltet.

12. System nach Anspruch 10, wobei das Kommunikationsteilsystem (606) ferner dazu konfiguriert ist, eine Annotationsvariable von der zweiten Benutzeroberfläche zu empfangen, wobei die Annotationsvariable eine Annotation zum Rendern auf der virtuellen zusammengesetzten Szene beschreibt; und
wobei das Videoteilsystem (602) ferner zu Folgendem konfiguriert ist:
Zusammensetzen der virtuellen zusammengesetzten Szene, um die Annotation zu beinhalten; und
Präsentieren der Annotation in der virtuellen zusammengesetzten Szene.

13. System nach Anspruch 10, wobei das Videoteilsystem (602) ferner zum Rendern der entfernten Szene als transluzente Schicht konfiguriert ist, wobei die transluzente Schicht dem Benutzer der ersten Benutzeroberfläche erlaubt, die lokale Szene zu betrachten, wenn er die virtuelle zusammengesetzte Szene betrachtet.

## Revendications

1. Procédé de gestion d'une interface utilisateur d'un système chirurgical télé-opéré, le procédé comprenant :
la réception (702) au niveau d'une première interface utilisateur (110) d'une variable environnementale, émise depuis une deuxième interface utilisateur et décrivant une opération d'un instrument virtuel éloigné dans la deuxième interface utilisateur, la variable environnementale étant une structure de données d'un ou de plusieurs n-uples, dans lequel l'instrument virtuel éloigné est opéré par un utilisateur de la deuxième interface utilisateur et la variable environnementale inclut une variable de commande de caméra ;
la réalisation d'un rendu (704) d'une scène locale au niveau de la première interface utilisateur (110) en utilisant la variable de commande de caméra, la scène locale représentant un état de fonctionnement de la première interface utilisateur, la scène locale représentant un site chirurgical virtuel incluant un instrument virtuel local, dans lequel l'instrument virtuel local est commandé par l'utilisateur de la première interface utilisateur ;
la réalisation d'un rendu (706) d'une scène éloignée au niveau de la première interface utilisateur (110), la scène éloignée représentant un état de fonctionnement de la deuxième interface utilisateur, la scène éloignée représentant le site chirurgical virtuel incluant l'instrument virtuel éloigné, dans lequel la scène éloignée est générée sur la base au moins en partie de la variable environnementale ;
la réalisation d'un composite (708) de la scène locale et de la scène éloignée pour produire une scène composite virtuelle ; et
la présentation (710) de la scène composite virtuelle à l'utilisateur de la première interface utilisateur (110), dans lequel la scène composite virtuelle inclut la scène locale et la scène éloignée, dans lequel la scène éloignée est modifiée en utilisant des manipulations graphiques pour distinguer l'instrument virtuel local de l'instrument virtuel éloigné.

2. Procédé selon la revendication 1, dans lequel la deuxième interface utilisateur inclut un dispositif de commande maître opéré par l'utilisateur de la deuxième interface utilisateur, et dans lequel la variable environnementale inclut une position, une vitesse, ou une rotation de l'opération du dispositif de commande maître.

3. Procédé selon la revendication 1, comprenant en outre :
la réception d'une variable d'annotation depuis la deuxième interface utilisateur, la variable d'annotation décrivant une annotation pour réaliser un rendu de la scène composite virtuelle ; et
la réalisation d'un composite de la scène composite virtuelle pour inclure l'annotation ;
dans lequel la présentation de la scène composite virtuelle inclut la présentation de l'annotation dans la scène composite virtuelle.

4. Procédé selon la revendication 3, dans lequel l'annotation inclut une commande de crayon, une commande de surligneur, ou un icone de pointeur.

5. Procédé selon la revendication 4, dans lequel l'annotation est sélectionnée par un utilisateur de la deuxième interface utilisateur.

6. Procédé selon la revendication 1, dans lequel l'instrument virtuel local est un instrument chirurgical virtuel.

7. Procédé selon la revendication 1, dans lequel la réalisation d'un rendu de la scène éloignée comprend la réalisation d'un rendu de la scène éloignée comme une couche translucide, la couche translucide permettant à l'utilisateur de la première interface utilisateur de visualiser la scène locale quand il visualise la scène composite virtuelle.

8. Procédé selon la revendication 1, comprenant en outre :
la fourniture d'un exercice chirurgical à l'utilisateur de la première interface utilisateur (110), et dans lequel l'exercice chirurgical est aussi fourni de manière substantiellement simultanée à un utilisateur de la deuxième interface utilisateur.

9. Procédé selon la revendication 1, dans lequel la réalisation d'un rendu de la scène locale et la réalisation d'un rendu de la scène éloignée sont effectuées sur des canevas différents.

10. Système destiné à gérer une interface utilisateur d'un système chirurgical télé-opéré, le système comprenant :
une première interface utilisateur (110) comprenant :
un sous-système de communication (606) configuré pour recevoir au niveau de la première interface utilisateur depuis une deuxième interface utilisateur, une variable environnementale décrivant une ou plusieurs opérations d'un instrument virtuel éloigné dans la deuxième interface utilisateur, la variable environnementale étant une structures de données d'un ou de plusieurs n-uples et dans lequel la variable environnementale inclut une variable de commande de caméra ; et
un sous-système de vidéo (602) configuré pour :
réaliser un rendu (704) d'une scène locale au niveau de la première interface utilisateur (110) en utilisant la variable de commande de caméra, la scène locale représentant un état de fonctionnement de la première interface utilisateur, la scène locale représentant un site chirurgical virtuel incluant un instrument virtuel local, et dans lequel l'instrument virtuel local est commandé par l'utilisateur de la première interface utilisateur ;
réaliser un rendu (706) d'une scène éloignée au niveau de la première interface utilisateur (110), la scène éloignée représentant un état de fonctionnement de la deuxième interface utilisateur, la scène éloignée représentant le site chirurgical virtuel incluant l'instrument virtuel éloigné, dans lequel la scène éloignée est générée sur la base au moins en partie de la variable environnementale ;
réaliser un composite (708) de la scène locale et la scène éloignée pour produire une scène composite virtuelle ; et
présenter (710) la scène composite virtuelle à un utilisateur de la première interface utilisateur (110), dans lequel la scène composite virtuelle inclut la scène locale et la scène éloignée, dans lequel la scène éloignée est modifiée en utilisant des manipulations graphiques pour distinguer l'instrument virtuel local de l'instrument virtuel éloigné.

11. Système selon la revendication 10, dans lequel la deuxième interface utilisateur inclut un dispositif de commande maître opéré par l'utilisateur de la deuxième interface utilisateur, et dans lequel la variable environnementale inclut une position, une vitesse, ou une rotation de l'opération du dispositif de commande maître.

12. Système selon la revendication 10, dans lequel le sous-système de communication (606) est configuré en outre pour recevoir une variable d'annotation depuis la deuxième interface utilisateur, la variable d'annotation décrivant une annotation pour réaliser un rendu sur la scène composite virtuelle ; et
dans lequel le sous-système de vidéo (602) est configuré en outre pour :
réaliser un composite de la scène composite virtuelle pour inclure l'annotation ; et
présenter l'annotation dans la scène composite virtuelle.

13. Système selon la revendication 10, dans lequel le sous-système vidéo (602) est configuré en outre pour réaliser un rendu de la scène éloignée comme une couche translucide, la couche translucide permettant à l'utilisateur de la première interface utilisateur de visualiser la scène locale quand il visualise la scène composite virtuelle.
